# DEMANDE DE BREVET EUROPEEN

(11) **EP 0 692 535 A1**
(43) Date de publication de la demande: **17.01.1996**
(21) Numéro de dépôt: 95401463.5
(22) Date de dépôt: 21.06.1995
(51) Int. Cl.: C12N 15/11, A61K 31/70, C07H 21/04

(54) **Oligonucléotides pour inhiber le rôle des isoprényl protéine transférases**

(30) Priorité: 29.06.1994 GB 9413035
(71) Demandeur: SOCIETE DE CONSEILS DE RECHERCHES ET D'APPLICATIONS SCIENTIFIQUES (S.C.R.A.S.), F-75016 Paris (FR)
(72) Inventeur: Colote, Soudhir, F-91940 Les Ulis (FR); Pirotzky, Eduardo, 75005 Paris (FR)
(74) Mandataire: Lachassagne, Jacques

(57) **Abrégé**

L'invention concerne des oligonucléotides et leurs dérivés pour inhiber le rôle des isoprényl protéine transférases, des compositions thérapeutiques les contenant et l'utilisation de telles compositions d'oligonucléotides pour le traitement ou thérapie des maladies du corps humain ou animal dans lesquelles la prolifération cellulaire est anormale et/ ou incontrôlée.

## Description

L'invention concerne des oligonucléotides et leurs dérivés pour inhiber le rôle des isoprényl protéine transférases, des compositions thérapeutiques contenant de tels composés et l'utilisation de telles compositions d'oligonucléotides pour le traitement ou la thérapie de maladies du corps humain ou animal, induites par une prolifération cellulaire anormale et / ou incontrôlée.

Tout organisme vivant, qu'il soit monocellulaire ou multicellulaire, se caractérise par son pouvoir à se multiplier, un phénomène nécessitant une réplication des matériels héréditaires (l'ADN, etc) et une répartition équitable de l'ADN répliqué entre les deux cellules "filles". Il existe un grand nombre de molécules très diverses, avec des activités mitogéniques, qui agissent de manière "cellules-spécifiques". Les molécules "signal" ou facteurs de croissance sont de type polypeptidique qui, en très faible quantité et après interaction avec des cellules cibles, peuvent provoquer une cascade d'événements qui aboutissent à la réplication de l'ADN et de la mitose.

Les facteurs de croissance n'agissent pas directement sur le cycle cellulaire mais par l'interaction et l'activation d'une série de récepteurs transmembranaires, localisés sur la membrane plasmique des cellules cibles. Ce mécanisme déclenche une réaction en chaîne, à l'intérieur de la cellule, pour aboutir à la mitose. Les événements intracellulaires activés peuvent varier d'un type cellulaire à l'autre pour un même récepteur.

L'une des premières étapes de liaison d'un facteur de croissance à son récepteur, est l'activation d'une protéine kinase et la phosphorylation des protéines. L'une des protéines qui constitue ce maillon de transduction de signal, est la protéine ras, une protéine de 21k-dalton, qui est modifiée par un mécanisme de prénylation. La prénylation est une modification post-traductionnelle qui consiste à lier, de façon covalente, des groupements isoprényles sur la cystéine de l'extrémité carboxyterminal de nombreuses protéines. Ainsi, l'isoprénylation peut être le transfert d'un groupement farnésyl ou d'un groupement géranylgéranyl sur les protéines cibles. Cette modification permet à certaines de ces protéines de s'accrocher à leur site d'action membranaire. Ainsi, une des conséquences d'inhibition de cette prénylation est d'empêcher la transduction des signaux extracellulaires vers le noyau et donc la prolifération cellulaire.

A ce jour, trois types d'enzymes qui catalysent cette modification ont été mis en évidence : la farnésyl-protéine transférase, la géranyl-géranyl-protéine transférase de type 1 et la géranyl-géranyl-protéine transférase de type 2. Toutes ces enzymes reconnaissent des séquences consensus situées à l'extrémité carboxy-terminale des protéines isoprényles.

L'une des conséquences de bloquer la synthèse de ces enzymes est le blocage des principales voies de transmission de signaux qui conduisent à la prolifération cellulaire.

La plupart des principes actifs classiques interagissent avec les protéines, produit de la traduction des informations génétiques, et inhibent leurs fonctions. La présente invention tend vers une approche thérapeutique plus récente: la stratégie antisens. Ladite approche vise à moduler sélectivement l'expression des gènes par une association très sélective d'un enchaînement de nucléotides (oligonucléotides) avec sa séquence complémentaire sur l'ARN messager, l'ARN pré-messager ou l'ADN et, donc, à inhiber la synthèse de la protéine correspondante. Les molécules utilisées ont une interaction directe sur la cascade d'informations génétiques.

Les oligonucléotides complémentaires aux produits de transcription sont appelés oligonucléotides "antisens". Les oligonucléotides ayant la même séquence que les produits de transcription sont nommés oligonucléotides "sens". Initialement, ces composés étaient logiquement destinés à inhiber la formation d'un produit du gène par la suppression de l'ARN messager correspondant, via le mécanisme d'hydrolyse catalysé par la RNAse H. Bientôt, il est apparu que le mécanisme d'action de ces oligonucléotides antisens n'était pas si simple. Ces oligonucléotides peuvent interagir avec un certain nombre de cibles cellulaires ne comprenant pas d'acide nucléique. Ces oligonucléotides peuvent interagir avec le gène, pour former des structures en triple hélice et inhiber la formation des produits de transcription. Les oligonucléotides peuvent interagir avec les jonctions intron-exon de l'ARN pre-messager, interférant ainsi avec l'épissage correct du produit de transcription. Les oligonucléotides peuvent s'hybrider avec l'ARN messager dans le cytoplasme, en formant un complexe ARN-ADN, qui est rapidement dégradé par l'enzyme ARNAse H ou en empêchant le complexe ribosome de se glisser sur l'ARN messager et bloquant ainsi la traduction. Les oligonucléotides et, plus particulièrement, les oligonucléotides modifiés, peuvent interagir avec un nombre de produits cellulaires tels que les protéines. Ces interactions peuvent être séquence-spécifiques (par exemple: facteurs de transcription) ou non-séquence-spécifiques (par exemple: facteurs de croissance). Ainsi, les oligonucléotides (SEQ ID Nos. 1-36) peuvent entraîner l'arrêt de la prolifération par l'un quelconque des mécanismes mentionnés ci-dessus ou une combinaison de ces mécanismes.

L'invention concerne plus particulièrement l'usage d'oligonucléotides antisens pour bloquer la synthèse d'enzymes telles que mentionnées ci-dessus. Ceci suggère leur utilisation en tant qu'agents anti-prolifératifs pour les maladies cardio-vasculaires, en cancérologie, en dermatologie, pour certaines infections virales et n'importe quelle pathologie qui implique une prolifération cellulaire.

Dans la méthodologie antisens, les oligonucléotides antisens ou les ARN antisens peuvent être utilisés. La méthodologie de l'oligonucléotide antisens est différente de l'ARN antisens. Dans cette dernière, un fragment de l'ADN d'un gène est inséré dans un ADN vecteur en sens opposé de son sens normal et, ainsi, lors de la transcription de ce dit vecteur, l'un des brins de son ADN est copié en ARN. L'insertion du fragment de gène à l'envers conduit à la synthèse *in situ* d'un ARN qui est complémentaire de l'ARN messager normal fabriqué par la cellule elle-même. Cet ARN, appelé ARN antisens, peut s'hybrider avec l'ARN messager normal et, ainsi, empêcher la traduction de la protéine cible. Une telle méthode a été utilisée par Prendergast et ses collaborateurs (Cell Growth and differenciation 4, 707-713, Septembre93), pour évaluer le rôle de la farnésyl protéine transférase: l'ADNc pour la sous-unité β de cette farnésyl protéine transférase est clonée et purifiée; les cellules cibles sont génétiquement modifiées pour exprimer *in situ* les molécules ARN antisens.

L'approche ARN antisens est particulièrement utile comme outil mécanistique pour élucider le rôle de la protéine dans une cellule et, éventuellement, comme agent thérapeutique, utilisant des protocoles de thérapie génique sophistiquée, alors que les oligonucléotides antisens sont plus attractifs en tant qu'agents pharmaceutiques et sont considérés par les autorités compétentes comme des entités chimiques classiques. De plus, les oligonucléotides peuvent être facilement synthétisés en grandes quantités par la chimie classique, alors que les molécules d'ARN antisens sont produites dans un système biologique et les difficultés pour les produire à l'échelle industrielle sont nombreuses.

Une méthode de dosage des farnésyl protéine transférases a été décrite dans la demande EP 456180: plus particulièrement, avec une telle méthode, l'activité d'un inhibiteur potentiel des farnésyl protéine transférases peut être mesurée. Mais, en aucun cas, cette méthode ne permet de définir la structure d'un nouvel inhibiteur potentiel des farnésyl protéine transférases ; par ailleurs, ce brevet ne décrit aucune molécule de la présente invention.

L'invention concerne des oligonucléotides antisens qui s'hybrident sélectivement avec un gène ou les produits de transcription des sous-unités des isoprényl protéine transférases et, de préférence, avec les sous-unités α et/ ou β des isoprényl protéine transférases. L'oligonucléotide peut comprendre de 2 à 50 unités et, de préférence, 8 à 35 unités. De manière très préférentielle, l'oligonucléotide comprend de 10 à 25 unités.

Les oligonucléotides antisens de l'invention peuvent être synthétisés par l'une quelconque des méthodes connues de synthèse chimique des oligonucléotides. Les oligonucléotides antisens sont très avantageusement préparés en utilisant n'importe quel synthétiseur automatique d'acide nucléique commercialisé. L'une de ces méthodes de synthèse des oligonucléotides est la méthode des beta-cyanoéthyl phosphoramidate décrite par S. L. Beaucage & ses collaborateurs (Tet. Let. 22 (1981), 1859-1862).

L'invention concerne également des dérivés de tels oligonucléotides, c'est à dire des oligonucléotides dont le squelette a été modifié soit sur toute la longueur de l'oligonucléotide, soit en position 5' et / ou en position 3'. En effet, les oligonucléotides sont sensibles à des enzymes, les nucléases qui les découpent en nucléotides; les oligonucléotides deviennent résistants aux nucléases par modification, par exemple, de la nature chimique du sucre lui-même ou l'enchaînement phosphate-sucre: ainsi, l'enchaînement phosphodiester peut être remplacé, par exemple, par un enchaînement phosphorothioate, phosphorodithioate, méthylphosphonate, phosphoramidate, phosphoéthyltriester, butylamidate, piperazidate ou morpholidate. D'autres types de modifications peuvent être apportés sur toute la longueur de l'oligonucléotide ou à ses extrémités 5' et/ ou 3', pour rendre les oligonucléotides plus résistants à un environnement biologique. Les liaisons phosphate entre les nucléotides peuvent être aussi remplacées par des liaisons amide (acides nucléiques peptidiques). Par ailleurs, le passage transmembranaire de l'oligonucléotide peut être favorisé en rendant ce dernier plus hydrophobe: ceci peut être obtenu, par exemple, en attachant des substituants hydrophobes tels que le cholestérol ou des groupements aromatiques, ou un polymère. Des bases modifiées peuvent être incorporées partiellement ou sur toute la longueur de l'oligonucléotide. Les nucléotides modifiés conformationnellement (par exemple, anon) résistants aux nucléases ou avec des propriétés améliorées d'absorption intracellulaire ou d'hybridation peuvent être incorporées partiellement ou sur toute la longueur de l'oligonucléotide. Ainsi, le terme "dérivé" comprend le nucléotide modifié selon l'une quelconque des méthodes décrites ci-dessus ou de toute autre méthode bien connue de l'homme du métier.

Les oligonucléotides préférés de l'invention sont les oligonucléotides des séquences respectives SEQ ID No. 1 à SEQ ID No. 36. Leurs séquences complémentaires ou les oligonucléotides sens selon l'invention, peuvent aussi être utilisées.

L'invention concerne également des oligonucléotides comprenant au moins un fragment de l'une des séquences sélectionnées parmi SEQ ID No. 1 à SEQ ID No. 36.

L'invention concerne également des compositions thérapeutiques contenant comme principe actif au moins un oligonucléotide antisens selon l'invention, mélangé avec un excipient et / ou support pharmaceutiquement acceptable selon le mode d'administration choisi. La composition peut être administrée pour un traitement topique ou systémique ou local;elle peut se présenter sous une forme liquide pour une injection, liposome, formulation à libération prolongée ou sous forme de gel, d'onguent pour une application locale ou sous toute autre forme acceptable selon le mode d'administration choisi.

Enfin, l'invention concerne l'utilisation d'une composition selon l'invention pour une méthode de traitement ou thérapie des maladies du corps humain ou animal dans lesquelles la prolifération cellulaire est anormale et / ou incontrôlée.

### Les Exemples ci-dessous illustrent l'invention.

### Exemple 1

### Effet des antisens sur la prolifération des muscles lisses de rat

Des cellules musculaires lisses d'aorte de rats mâles Wistar sont isolées par digestion enzymatique (collagénase et élastase) et cultivées en présence de milieu DMEM, 10 % de sérum de veau foetal (FCS), 2 mM de glutamine, 50 U/ml de pénicilline et 50 µg/ml de streptomycine. Les cellules musculaires lisses sont utilisées entre le 3^{ème} et 7^{ème} passage dans les plaques 24 puits.

Après 3 jours de culture, les cellules sont incubées dans un milieu sans sérum pendant 72 heures. Les cellules sont ensuite stimulées (ou non) avec 1 % de sérum ou 5 ng/ml de bFGF (basic Fibroblast Growth Factor) et traitées simultanément avec ou sans les produits de l'invention, à la concentration de 10⁻⁵ M, pendant 24 heures. Quatre heures avant la fin de cette incubation, les cellules sont marquées à la thymidine tritiée (1 µCi/ml). L'incorporation est arrêtée par addition de TCA 5 %, pendant 10 minutes ; 500 µl de NaOH 0,5 N sont ajoutés aux cellules. Des aliquotes de 400 µl sont prélevés et distribués dans des flacons à scintillation contenant 400 µl d'HCl 0,5 N et 10 ml d'Instagel.

L'activité des oligonucléotides est déterminée par la mesure de la prolifération cellulaire, exprimée en pourcentage. Les résultats obtenus sont regroupés dans le tableau ci-dessous.

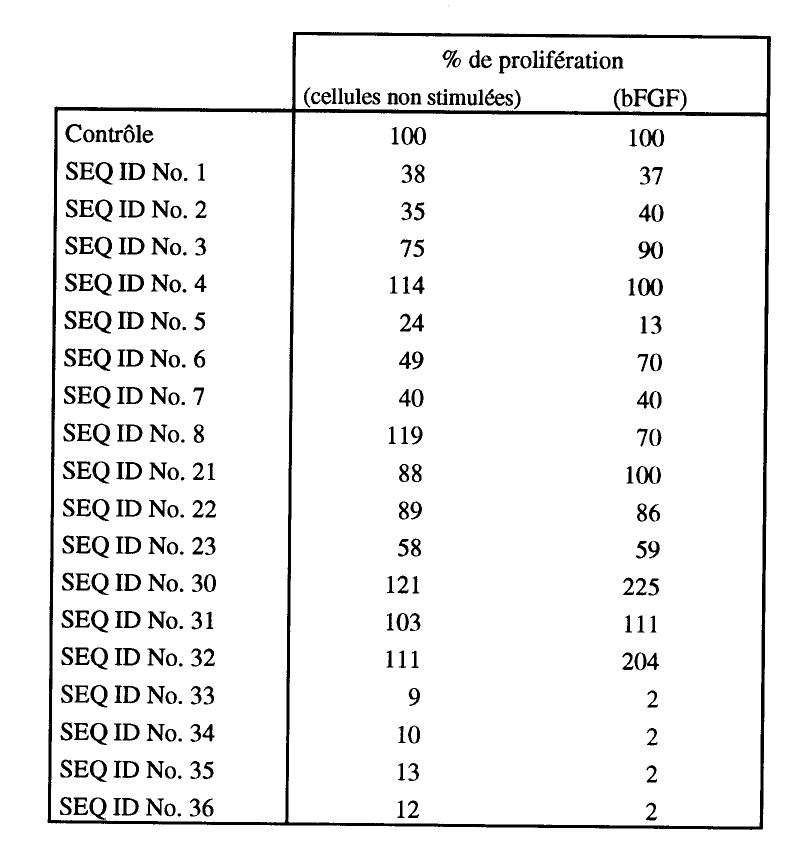

### Exemple 2

### Etude dose-réponse - Effet des antisens sur la prolifération des muscles lisses de rat

Le protocole expérimental est identique à celui décrit dans l'Exemple 1. Les cellules sont traitées avec les composés de l'invention, à des concentrations de 10⁻⁹M, 10⁻⁸M, 10⁻⁷M, 10⁻⁶M et 10⁻⁵M.

| Concentration | % de prolifération | | | | | |
|---|---|---|---|---|---|---|
| | 0 | 10⁻⁹M | 10⁻⁸M | 10⁻⁷M | 10⁻⁶M | 10⁻⁵M |
| SEQ ID No. 1 | 100 | 99 | 108 | 107 | 64 | 25 |
| SEQ ID No. 2 | 100 | 86 | 81 | 90 | 50 | 14 |
| SEQ ID No. 5 | 100 | 95 | 80 | 100 | 66 | 20 |
| SEQ ID No. 6 | 100 | 87 | 69 | 84 | 48 | 20 |
| SEQ ID No. 7 | 100 | 102 | 98 | 96 | 59 | 28 |

### Exemple 3

### Effet de la taille sur l'activité biologique in vitro des oligonucléotides antisens

Le protocole expérimental est identique à celui décrit dans l'Exemple 1. Les cellules sont traitées avec les composés de l'invention.

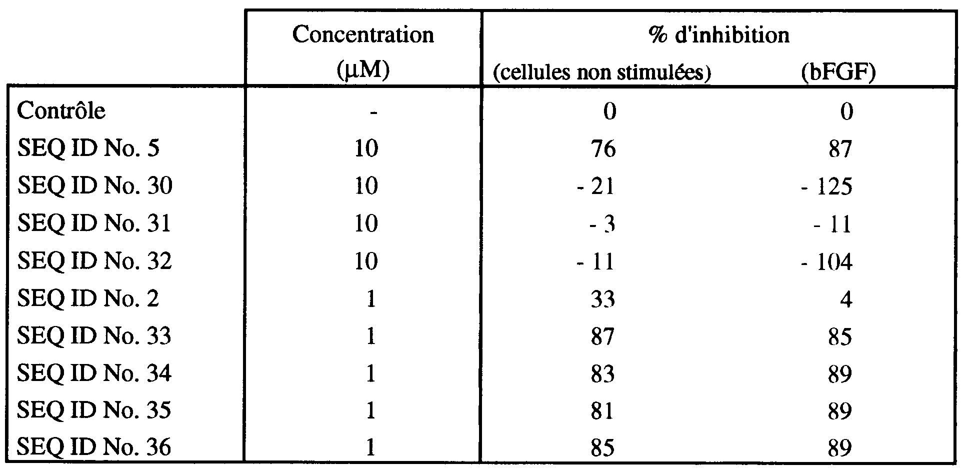

### Exemple 4

### Etudes de l'activité antiproliférative in vivo des oligonucléotides antisens sur un modèle d'angioplastie (rat ou lapin)

Des rats Wistar mâles normotensive ou des lapins Nouvelle-Zélande sont anesthésiés et disséqués pour isoler l'aorte ou l'artère iliaque. Après aortotomie, un cathéter French Fogarty est introduit et le ballon gonflé avec 0,2 ml d'une solution saline. La désendothélialisation a été effectuée par 3 passages du ballonnet gonflé.

L'oligonucléotide solubilisé dans 25 % de gel pluronique est immédiatement appliqué pour recouvrir la partie isolée de l'artère. Les plaies sont refermées et les animaux sont sacrifiés 21 jours plus tard. L'analyse histomorphométrique des sections de l'artère désendothélialisée est effectuée et les surfaces intima et médianes sont mesurées.

Le pourcentage de prolifération est défini par le rapport intima / intima + médiane.

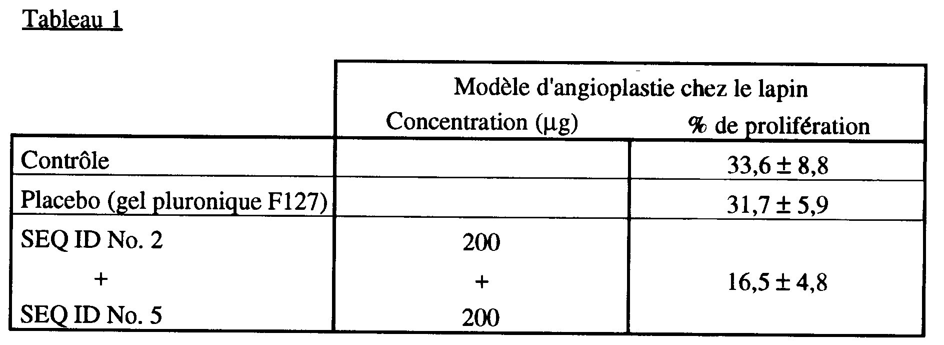

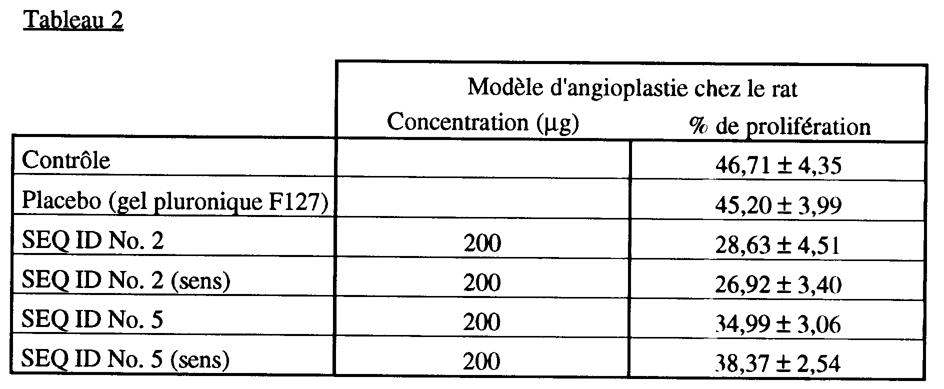

Par convention, tout au long de la description, l'expression "SEQ ID (sens)" est la séquence oligonucléotidique complémentaire de la séquence oligonucléotidique SEQ ID.

### Exemple 5

### a) Effet in vitro des oligonucléotides sens et antisens pour la farnésyl transférase sur des cellules gliales de rat C6

Les cellules sont ensemencées à la densité de 5000 cellules / boîte et incubées avec un milieu complété avec 5 % de sérum de cheval, 2,5 % de sérum de veau foetal. 24 heures plus tard, le milieu est remplacé par un milieu sans sérum contenant en plus de la lipofectine et les différents oligonucléotides, à la concentration de 5 µM, pour réaliser les transfections. Après 5 heures d'incubation, le milieu est remplacé par un milieu normal, contenant 15 % de sérum de cheval et 2,5 % de sérum de veau foetal. L'incubation est ainsi maintenue pendant 72 heures, à 37° C. La prolifération cellulaire est évaluée par le dénombrement des cellules, après trypsination de la monocouche, en utilisant un compteur Coulter Counter model ZM.

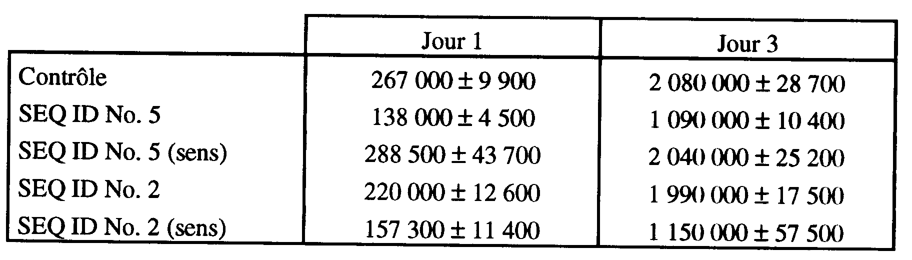

### b) Effet "time course" des oligonucléotides pour la sous-unité α de la farnésyl transférase

Les cellules sont traitées comme décrit ci-dessus et le dénombrement des cellules est fait sur des échantillons en parallèle du contrôle et sur des plateaux traités par les oligonucléotides aux jours 3, 7 et 14. La valeur p est inférieure à 0,05 % pendant les 3 jours de l'expérience.

| Jour | Contrôle (nombre de cellules) | SEQ ID No. 5 (5 µM) (nombre de cellules) |
|---|---|---|
| 0 | 5 000 | 5 000 |
| 3 | 950 000 | 350 000 |
| 7 | 2 100 000 | 600 000 |
| 14 | 3 750 000 | 1 350 000 |

### Exemple 6

### Etudes antiprolifératives sur le gliome chez le rat

### a) Effet ex vivo des oligonucléotides antisens sur la tumeur gliale (cellules C6)

Les cellules sont traitées tel que décrit ci-dessus. 24 heures après la transfection avec l'antisens de la sous-unité α pour la farnésyl transférase, les cellules antisens (5 µM) et les cellules du contrôle sont inoculées intracérébralement chez des rats mâles Sprague-Dawley (âgés de 7 à 8 semaines et d'un poids de 180 à 200 g). Les animaux sont anesthésiés par une injection i.p. de 1,5 mg/kg de xilazine, suivie par une injection i.p. de 5 mg/kg de kétamine après 10 minutes. 1,5 x 10⁵ cellules dans 3 µl de solution saline tamponnée au phosphate, préalablement récupérées par trypsination, sont injectées dans le ganglion basal gauche d'après les coordonnées stéréotaxiques suivantes : à 3 mm de la ligne médiane et à une profondeur de 5 mm, en utilisant une microseringue Hamilton de 10 µl. Au préalable, il a été observé chez les rats qui ont été sacrifiés les jours suivant l'implantation de la tumeur que le taux de survie moyen est de 24 jours. L'analyse histologique des cerveaux effectuée pour tous les animaux révèle que l'implantation de la tumeur a réussi à 100 %. 2 heures avant le sacrifice, 40 mg de BrdU sont injectés chez tous les rats. Les animaux sont décapités. Tous les cerveaux sont prélevés chez tous les animaux et fixés par immersion dans de l'éthanol à 70 %, pendant 2 jours, à 4° C. La prolifération de la tumeur est définie par les moyennes de l'analyse cytofluorimétrique ADN biparamétrique (BrdU) des deux hémisphères du cerveau.

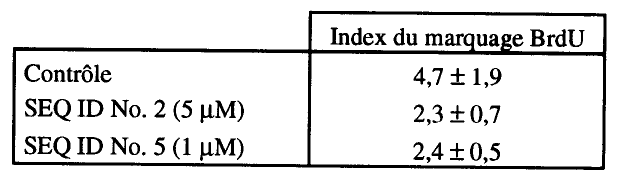

### b) Effet in vivo des oligonucléotides sur la tumorigénicité in vivo de cellules gliales C6

Les cellules gliales C6 non traitées sont placées 48 heures dans des boîtes de culture cellulaire, puis injectées dans l'hémisphère gauche des cerveaux de rat, tel que décrit ci-dessus. Immédiatement après l'injection cellulaire, une pompe Alzet contenant soit le placebo, soit l'oligonucléotide sens et antisens (en quantité suffisante pour délivrer une concentration d'oligonucléotides de 5 µM par jour), est placée sur une poche dorsale. Différents agents sont délivrés sur le site de l'injection des cellules, au moyen d'un tuyau de 0,5 mm de diamètre. Toutes les analyses statistiques sont réalisées en utilisant le test Duncan.

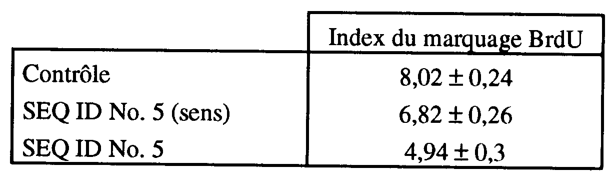

LISTE DES SEQUENCES

(2) INFORMATIONS CONCERNANT SEQ ID No. 1

(i) CARACTERISTIQUES DE LA SEQUENCE :
(A) LONGUEUR : 12 paires de base
(B) TYPE : nucléotide
(C) NOMBRE DE BRINS : simple
(D) CONFIGURATION : linéaire

(iv) ANTI-SENS : Oui

(xi) DESCRIPTION DE LA SEQUENCE : SEQ ID No. 1
AGAAGCCATG AT

(2) INFORMATIONS CONCERNANT SEQ ID No. 2

(i) CARACTERISTIQUES DE LA SEQUENCE :
(A) LONGUEUR : 12 paires de base
(B) TYPE : nucléotide
(C) NOMBRE DE BRINS : simple
(D) CONFIGURATION : linéaire

(iv) ANTI-SENS : Oui

(xi) DESCRIPTION DE LA SEQUENCE : SEQ ID No. 2
GGACGTGACT GT

(2) INFORMATIONS CONCERNANT SEQ ID No. 3

(i) CARACTERISTIQUES DE LA SEQUENCE :
(A) LONGUEUR : 12 paires de base
(B) TYPE : nucléotide
(C) NOMBRE DE BRINS : simple
(D) CONFIGURATION : linéaire

(iv) ANTI-SENS : Oui

(xi) DESCRIPTION DE LA SEQUENCE : SEQ ID No. 3
AAGGAGTAGC AG

(2) INFORMATIONS CONCERNANT SEQ ID No. 4

(i) CARACTERISTIQUES DE LA SEQUENCE :
(A) LONGUEUR : 12 paires de base
(B) TYPE : nucléotide
(C) NOMBRE DE BRINS : simple
(D) CONFIGURATION : linéaire

(iv) ANTI-SENS : Oui

(xi) DESCRIPTION DE LA SEQUENCE : SEQ ID No. 4
CAGGCAGTAG CA

(2) INFORMATIONS CONCERNANT SEQ ID No. 5

(i) CARACTERISTIQUES DE LA SEQUENCE :
(A) LONGUEUR : 12 paires de base
(B) TYPE : nucléotide
(C) NOMBRE DE BRINS : simple
(D) CONFIGURATION : linéaire

(iv) ANTI-SENS : Oui

(xi) DESCRIPTION DE LA SEQUENCE : SEQ ID No. 5
CCCGTCGTCC AT

(2) INFORMATIONS CONCERNANT SEQ ID No. 6

(i) CARACTERISTIQUES DE LA SEQUENCE :
(A) LONGUEUR : 12 paires de base
(B) TYPE : nucléotide
(C) NOMBRE DE BRINS : simple
(D) CONFIGURATION : linéaire

(iv) ANTI-SENS : Oui

(xi) DESCRIPTION DE LA SEQUENCE : SEQ ID No. 6
CTGTCCCTGT AC

(2) INFORMATIONS CONCERNANT SEQ ID No. 7

(i) CARACTERISTIQUES DE LA SEQUENCE :
(A) LONGUEUR : 12 paires de base
(B) TYPE : nucléotide
(C) NOMBRE DE BRINS : simple
(D) CONFIGURATION : linéaire

(iv) ANTI-SENS : Oui

(xi) DESCRIPTION DE LA SEQUENCE : SEQ ID No. 7
ACTTCTCTCT CC

(2) INFORMATIONS CONCERNANT SEQ ID No. 8

(i) SEQUENCE CHARACTERISTICS :

(i) CARACTERISTIQUES DE LA SEQUENCE :
(A) LONGUEUR : 12 paires de base
(B) TYPE : nucléotide
(C) NOMBRE DE BRINS : simple
(D) CONFIGURATION : linéaire

(iv) ANTI-SENS : Oui

(xi) DESCRIPTION DE LA SEQUENCE : SEQ ID No. 8
ACTTGGTCCT AA

(2) INFORMATIONS CONCERNANT SEQ ID No. 9

(i) CARACTERISTIQUES DE LA SEQUENCE :
(A) LONGUEUR : 12 paires de base
(B) TYPE : nucléotide
(C) NOMBRE DE BRINS : simple
(D) CONFIGURATION : linéaire

(iv) ANTI-SENS : Oui

(xi) DESCRIPTION DE LA SEQUENCE : SEQ ID No. 9
GCCATCATTC TG

(2) INFORMATIONS CONCERNANT SEQ ID No. 10

(i) CARACTERISTIQUES DE LA SEQUENCE :
(A) LONGUEUR : 12 paires de base
(B) TYPE : nucléotide
(C) NOMBRE DE BRINS : simple
(D) CONFIGURATION : linéaire

(iv) ANTI-SENS : Oui

(xi) DESCRIPTION DE LA SEQUENCE : SEQ ID No. 10
GATCTGGACC AC

(2) INFORMATIONS CONCERNANT SEQ ID No. 11

(i) SEQUENCE CHARACTERISTICS :

(i) CARACTERISTIQUES DE LA SEQUENCE :
(A) LONGUEUR : 12 paires de base
(B) TYPE : nucléotide
(C) NOMBRE DE BRINS : simple
(D) CONFIGURATION : linéaire

(iv) ANTI-SENS : Oui

(xi) DESCRIPTION DE LA SEQUENCE : SEQ ID No. 11
CTCAATAGCA TC

(2) INFORMATIONS CONCERNANT SEQ ID No. 12

(i) CARACTERISTIQUES DE LA SEQUENCE :
(A) LONGUEUR : 12 paires de base
(B) TYPE : nucléotide
(C) NOMBRE DE BRINS : simple
(D) CONFIGURATION : linéaire

(iv) ANTI-SENS : Oui

(xi) DESCRIPTION DE LA SEQUENCE : SEQ ID No. 12
GTTTTTGGGC TG

(2) INFORMATIONS CONCERNANT SEQ ID No. 13

(i) CARACTERISTIQUES DE LA SEQUENCE :
(A) LONGUEUR : 12 paires de base
(B) TYPE : nucléotide
(C) NOMBRE DE BRINS : simple
(D) CONFIGURATION : linéaire

(iv) ANTI-SENS : Oui

(xi) DESCRIPTION DE LA SEQUENCE : SEQ ID No. 13
TCTCACATCC TC

(2) INFORMATIONS CONCERNANT SEQ ID No. 14

(i) CARACTERISTIQUES DE LA SEQUENCE :
(A) LONGUEUR : 12 paires de base
(B) TYPE : nucléotide
(C) NOMBRE DE BRINS : simple
(D) CONFIGURATION : linéaire

(iv) ANTI-SENS : Oui

(xi) DESCRIPTION DE LA SEQUENCE : SEQ ID No. 14
TTGTAAGAAC TG

(2) INFORMATIONS CONCERNANT SEQ ID No. 15

(i) CARACTERISTIQUES DE LA SEQUENCE :
(A) LONGUEUR : 12 paires de base
(B) TYPE : nucléotide
(C) NOMBRE DE BRINS : simple
(D) CONFIGURATION : linéaire

(iv) ANTI-SENS : Oui

(xi) DESCRIPTION DE LA SEQUENCE : SEQ ID No. 15
GAAGTGCTTC TC

(2) INFORMATIONS CONCERNANT SEQ ID No. 16
(i) CARACTERISTIQUES DE LA SEQUENCE :
(A) LONGUEUR : 12 paires de base
(B) TYPE : nucléotide
(C) NOMBRE DE BRINS : simple
(D) CONFIGURATION : linéaire
(iv) ANTI-SENS : Oui
(xi) DESCRIPTION DE LA SEQUENCE : SEQ ID No. 16
   GCCTCTTTTC AG
(2) INFORMATIONS CONCERNANT SEQ ID No. 17
(i) CARACTERISTIQUES DE LA SEQUENCE :
(A) LONGUEUR : 12 paires de base
(B) TYPE : nucléotide
(C) NOMBRE DE BRINS : simple
(D) CONFIGURATION : linéaire
(iv) ANTI-SENS : Oui
(xi) DESCRIPTION DE LA SEQUENCE : SEQ ID No. 17
GGCATCTGTC AG
(2) INFORMATIONS CONCERNANT SEQ ID No. 18
(i) CARACTERISTIQUES DE LA SEQUENCE :
(A) LONGUEUR : 12 paires de base
(B) TYPE : nucléotide
(C) NOMBRE DE BRINS : simple
(D) CONFIGURATION : linéaire
(iv) ANTI-SENS : Oui
(xi) DESCRIPTION DE LA SEQUENCE : SEQ ID No. 18
CGGCTGGCAT CC

(2) INFORMATIONS CONCERNANT SEQ ID No. 19

(i) CARACTERISTIQUES DE LA SEQUENCE :
(A) LONGUEUR : 12 paires de base
(B) TYPE : nucléotide
(C) NOMBRE DE BRINS : simple
(D) CONFIGURATION : linéaire

(iv) ANTI-SENS : Oui

(xi) DESCRIPTION DE LA SEQUENCE : SEQ ID No. 19
GAACTGACAC AC

(2) INFORMATIONS CONCERNANT SEQ ID No. 20

(i) CARACTERISTIQUES DE LA SEQUENCE :
(A) LONGUEUR : 12 paires de base
(B) TYPE : nucléotide
(C) NOMBRE DE BRINS : simple
(D) CONFIGURATION : linéaire

(iv) ANTI-SENS : Oui

(xi) DESCRIPTION DE LA SEQUENCE : SEQ ID No. 20
ACGATCACAT CA

(2) INFORMATIONS CONCERNANT SEQ ID No. 21

(i) CARACTERISTIQUES DE LA SEQUENCE :
(A) LONGUEUR : 12 paires de base
(B) TYPE : nucléotide
(C) NOMBRE DE BRINS : simple
(D) CONFIGURATION : linéaire

(iv) ANTI-SENS : Oui

(xi) DESCRIPTION DE LA SEQUENCE : SEQ ID No. 21
ATCAACATGC AG

(2) INFORMATIONS CONCERNANT SEQ ID No. 22

(i) CARACTERISTIQUES DE LA SEQUENCE :
(A) LONGUEUR : 12 paires de base
(B) TYPE : nucléotide
(C) NOMBRE DE BRINS : simple
(D) CONFIGURATION : linéaire

(iv) ANTI-SENS : Oui

(xi) DESCRIPTION DE LA SEQUENCE : SEQ ID No. 22
CTGCATATCG AC

(2) INFORMATIONS CONCERNANT SEQ ID No. 23

(i) CARACTERISTIQUES DE LA SEQUENCE :
(A) LONGUEUR : 12 paires de base
(B) TYPE : nucléotide
(C) NOMBRE DE BRINS : simple
(D) CONFIGURATION : linéaire

(iv) ANTI-SENS : Oui

(xi) DESCRIPTION DE LA SEQUENCE : SEQ ID No. 23
CAGTGCACCA GC

(2) INFORMATIONS CONCERNANT SEQ ID No. 24

(i) CARACTERISTIQUES DE LA SEQUENCE :
(A) LONGUEUR : 12 paires de base
(B) TYPE : nucléotide
(C) NOMBRE DE BRINS : simple
(D) CONFIGURATION : linéaire

(iv) ANTI-SENS : Oui

(xi) DESCRIPTION DE LA SEQUENCE : SEQ ID No. 24
CTGCAGCCTC GG

(2) INFORMATIONS CONCERNANT SEQ ID No. 25

(i) CARACTERISTIQUES DE LA SEQUENCE :
(A) LONGUEUR : 12 paires de base
(B) TYPE : nucléotide
(C) NOMBRE DE BRINS : simple
(D) CONFIGURATION : linéaire

(iv) ANTI-SENS : Oui

(xi) DESCRIPTION DE LA SEQUENCE : SEQ ID No. 25
ACTCTCTGGA CC

(2) INFORMATIONS CONCERNANT SEQ ID No. 26

(i) CARACTERISTIQUES DE LA SEQUENCE :
(A) LONGUEUR : 12 paires de base
(B) TYPE : nucléotide
(C) NOMBRE DE BRINS : simple
(D) CONFIGURATION : linéaire

(iv) ANTI-SENS : Oui

(xi) DESCRIPTION DE LA SEQUENCE : SEQ ID No. 26
CAAGAATCCT CG

(2) INFORMATIONS CONCERNANT SEQ ID No. 27

(i) CARACTERISTIQUES DE LA SEQUENCE :
(A) LONGUEUR : 12 paires de base
(B) TYPE : nucléotide
(C) NOMBRE DE BRINS : simple
(D) CONFIGURATION : linéaire

(iv) ANTI-SENS : Oui

(xi) DESCRIPTION DE LA SEQUENCE : SEQ ID No. 27
ACATCTGCTC TG

(2) INFORMATIONS CONCERNANT SEQ ID No. 28
(i) CARACTERISTIQUES DE LA SEQUENCE :
(A) LONGUEUR : 12 paires de base
(B) TYPE : nucléotide
(C) NOMBRE DE BRINS : simple
(D) CONFIGURATION : linéaire
(iv) ANTI-SENS : Oui
(xi) DESCRIPTION DE LA SEQUENCE : SEQ ID No. 28
TCTGTACTCG TC
(2) INFORMATIONS CONCERNANT SEQ ID No. 29
(i) CARACTERISTIQUES DE LA SEQUENCE :
(A) LONGUEUR : 12 paires de base
(B) TYPE : nucléotide
(C) NOMBRE DE BRINS : simple
(D) CONFIGURATION : linéaire
(iv) ANTI-SENS : Oui
(xi) DESCRIPTION DE LA SEQUENCE : SEQ ID No. 29
CTACAGTACA GC
(2) INFORMATIONS CONCERNANT SEQ ID No. 30
(i) CARACTERISTIQUES DE LA SEQUENCE :
(A) LONGUEUR : 15 paires de base
(B) TYPE : nucléotide
(C) NOMBRE DE BRINS : simple
(D) CONFIGURATION : linéaire
(iv) ANTI-SENS : Oui
(xi) DESCRIPTION DE LA SEQUENCE : SEQ ID No. 30
CCCGTCGTCC ATAGG

(2) INFORMATIONS CONCERNANT SEQ ID No. 31

(i) CARACTERISTIQUES DE LA SEQUENCE :
(A) LONGUEUR : 18 paires de base
(B) TYPE : nucléotide
(C) NOMBRE DE BRINS : simple
(D) CONFIGURATION : linéaire

(iv) ANTI-SENS : Oui

(xi) DESCRIPTION DE LA SEQUENCE : SEQ ID No. 31
CCCGTCGTCC ATAGGGGA

(2) INFORMATIONS CONCERNANT SEQ ID No. 32

(i) CARACTERISTIQUES DE LA SEQUENCE :
(A) LONGUEUR : 21 paires de base
(B) TYPE : nucléotide
(C) NOMBRE DE BRINS : simple
(D) CONFIGURATION : linéaire

(iv) ANTI-SENS : Oui

(xi) DESCRIPTION DE LA SEQUENCE : SEQ ID No. 32
CCCGTCGTCC ATAGGGGACG C

(2) INFORMATIONS CONCERNANT SEQ ID No. 33

(i) CARACTERISTIQUES DE LA SEQUENCE :
(A) LONGUEUR : 15 paires de base
(B) TYPE : nucléotide
(C) NOMBRE DE BRINS : simple
(D) CONFIGURATION : linéaire

(iv) ANTI-SENS : Oui

(xi) DESCRIPTION DE LA SEQUENCE : SEQ ID No. 33
GGACGTGACT GTTTC

(2) INFORMATIONS CONCERNANT SEQ ID No. 34
(i) CARACTERISTIQUES DE LA SEQUENCE :
(A) LONGUEUR : 18 paires de base
(B) TYPE : nucléotide
(C) NOMBRE DE BRINS : simple
(D) CONFIGURATION : linéaire
(iv) ANTI-SENS : Oui
(xi) DESCRIPTION DE LA SEQUENCE : SEQ ID No. 34
GGACGTGACT GTTTCCAC
(2) INFORMATIONS CONCERNANT SEQ ID No. 35
(i) CARACTERISTIQUES DE LA SEQUENCE :
(A) LONGUEUR : 21 paires de base
(B) TYPE : nucléotide
(C) NOMBRE DE BRINS : simple
(D) CONFIGURATION : linéaire
(iv) ANTI-SENS : Oui
(xi) DESCRIPTION DE LA SEQUENCE : SEQ ID No. 35
GGACGTGACT GTTTCCACTG A
(2) INFORMATIONS CONCERNANT SEQ ID No. 36
(i) CARACTERISTIQUES DE LA SEQUENCE :
(A) LONGUEUR : 24 paires de base
(B) TYPE : nucléotide
(C) NOMBRE DE BRINS : simple
(D) CONFIGURATION : linéaire
(iv) ANTI-SENS : Oui
(xi) DESCRIPTION DE LA SEQUENCE : SEQ ID No. 36
GGACGTGACT GTTTCCACTG AGTC

LISTE DE SEQUENCES

(1) INFORMATIONS GENERALES:

(i) DEPOSANT:
(A) NOM: SOCIETE DE CONSEILS DE RECHERCHES ET
D'APPLICATIONS SCIENTIFIQUES (SCRAS)
(B) RUE: 51/53 RUE DU DOCTEUR BLANCHE
(C) VILLE: PARIS
(E) PAYS: FRANCE
(F) CODE POSTAL: 75016

(ii) TITRE DE L' INVENTION: OLIGONUCLEOTIDES POUR INHIBER LE ROLE DES
ISOPRENYL PROTEINE TRANSFERASES

(iii) NOMBRE DE SEQUENCES: 36

(iv) FORME DECHIFFRABLE PAR ORDINATEUR:
(A) TYPE DE SUPPORT: Floppy disk
(B) ORDINATEUR: IBM PC compatible
(C) SYSTEME D' EXPLOITATION: PC-DOS/MS-DOS
(D) LOGICIEL: PatentIn Release #1.0, Version #1.30 (OEB)

(vi) DONNEES DE LA DEMANDE ANTERIEURE:
(A) NUMERO DE LA DEMANDE: GB 9413035.8
(B) DATE DE DEPOT: 29-JUN-1994

(2) INFORMATIONS POUR LA SEQ ID NO: 1:

(i) CARACTERISTIQUES DE LA SEQUENCE:
(A) LONGUEUR: 12 paires de bases
(B) TYPE: nucléotide
(C) NOMBRE DE BRINS: simple
(D) CONFIGURATION: linéaire

(iv) ANTI-SENS: OUI

(xi) DESCRIPTION DE LA SEQUENCE: SEQ ID NO: 1:

AGAAGCCATG AT 12

(2) INFORMATIONS POUR LA SEQ ID NO: 2:

(i) CARACTERISTIQUES DE LA SEQUENCE:
(A) LONGUEUR: 12 paires de bases
(B) TYPE: nucléotide
(C) NOMBRE DE BRINS: simple
(D) CONFIGURATION: linéaire

(iv) ANTI-SENS: OUI

(xi) DESCRIPTION DE LA SEQUENCE: SEQ ID NO: 2:

GGACGTGACT GT                                                    12

(2) INFORMATIONS POUR LA SEQ ID NO: 3:

(i) CARACTERISTIQUES DE LA SEQUENCE:
(A) LONGUEUR: 12 paires de bases
(B) TYPE: nucléotide
(C) NOMBRE DE BRINS: simple
(D) CONFIGURATION: linéaire

(iv) ANTI-SENS: OUI

(xi) DESCRIPTION DE LA SEQUENCE: SEQ ID NO: 3:

[?]AGGAGTAGC AG                                                    12

(2) INFORMATIONS POUR LA SEQ ID NO: 4:

(i) CARACTERISTIQUES DE LA SEQUENCE:
(A) LONGUEUR: 12 paires de bases
(B) TYPE: nucléotide
(C) NOMBRE DE BRINS: simple
(D) CONFIGURATION: linéaire

(iv) ANTI-SENS: OUI

(xi) DESCRIPTION DE LA SEQUENCE: SEQ ID NO: 4:

[?]AGGCAGTAG CA                                                    12

(2) INFORMATIONS POUR LA SEQ ID NO: 5:

(i) CARACTERISTIQUES DE LA SEQUENCE:
(A) LONGUEUR: 12 paires de bases
(B) TYPE: nucléotide
(C) NOMBRE DE BRINS: simple
(D) CONFIGURATION: linéaire

(iv) ANTI-SENS: OUI

(xi) DESCRIPTION DE LA SEQUENCE: SEQ ID NO: 5:

CCCGTCGTCC AT                                                    12

(2) INFORMATIONS POUR LA SEQ ID NO: 6:

(i) CARACTERISTIQUES DE LA SEQUENCE:
(A) LONGUEUR: 12 paires de bases
(B) TYPE: nucléotide
(C) NOMBRE DE BRINS: simple
(D) CONFIGURATION: linéaire

(iv) ANTI-SENS: OUI

(xi) DESCRIPTION DE LA SEQUENCE: SEQ ID NO: 6:

CTGTCCCTGT AC 12

(2) INFORMATIONS POUR LA SEQ ID NO: 7:

(i) CARACTERISTIQUES DE LA SEQUENCE:
(A) LONGUEUR: 12 paires de bases
(B) TYPE: nucléotide
(C) NOMBRE DE BRINS: simple
(D) CONFIGURATION: linéaire

(iv) ANTI-SENS: OUI

(xi) DESCRIPTION DE LA SEQUENCE: SEQ ID NO: 7:

ACTTCTCTCT CC 12

(2) INFORMATIONS POUR LA SEQ ID NO: 8:

(i) CARACTERISTIQUES DE LA SEQUENCE:
(A) LONGUEUR: 12 paires de bases
(B) TYPE: nucléotide
(C) NOMBRE DE BRINS: simple
(D) CONFIGURATION: linéaire

(iv) ANTI-SENS: OUI

(xi) DESCRIPTION DE LA SEQUENCE: SEQ ID NO: 8:

ACTTGGTCCT AA 12

(2) INFORMATIONS POUR LA SEQ ID NO: 9:

(i) CARACTERISTIQUES DE LA SEQUENCE:
(A) LONGUEUR: 12 paires de bases
(B) TYPE: nucléotide
(C) NOMBRE DE BRINS: simple

(D) CONFIGURATION: linéaire

(iv) ANTI-SENS: OUI

(xi) DESCRIPTION DE LA SEQUENCE: SEQ ID NO: 9:

GCCATCATTC TG 12

(2) INFORMATIONS POUR LA SEQ ID NO: 10:

(i) CARACTERISTIQUES DE LA SEQUENCE:
(A) LONGUEUR: 12 paires de bases
(B) TYPE: nucléotide
(C) NOMBRE DE BRINS: simple
(D) CONFIGURATION: linéaire

(iv) ANTI-SENS: OUI

(xi) DESCRIPTION DE LA SEQUENCE: SEQ ID NO: 10:

GATCTGGACC AC 12

(2) INFORMATIONS POUR LA SEQ ID NO: 11:

(i) CARACTERISTIQUES DE LA SEQUENCE:
(A) LONGUEUR: 12 paires de bases
(B) TYPE: nucléotide
(C) NOMBRE DE BRINS: simple
(D) CONFIGURATION: linéaire

(iv) ANTI-SENS: OUI

(xi) DESCRIPTION DE LA SEQUENCE: SEQ ID NO: 11:

CTCAATAGCA TC 12

(2) INFORMATIONS POUR LA SEQ ID NO: 12:

(i) CARACTERISTIQUES DE LA SEQUENCE:
(A) LONGUEUR: 12 paires de bases
(B) TYPE: nucléotide
(C) NOMBRE DE BRINS: simple
(D) CONFIGURATION: linéaire

(iv) ANTI-SENS: OUI

(xi) DESCRIPTION DE LA SEQUENCE: SEQ ID NO: 12:

GTTTTTGGGC TG 12

(2) INFORMATIONS POUR LA SEQ ID NO: 13:

(i) CARACTERISTIQUES DE LA SEQUENCE:
(A) LONGUEUR: 12 paires de bases
(B) TYPE: nucléotide
(C) NOMBRE DE BRINS: simple
(D) CONFIGURATION: linéaire

(iv) ANTI-SENS: OUI

(xi) DESCRIPTION DE LA SEQUENCE: SEQ ID NO: 13:

[?]TCACATCC TC 12

(2) INFORMATIONS POUR LA SEQ ID NO: 14:

(i) CARACTERISTIQUES DE LA SEQUENCE:
(A) LONGUEUR: 12 paires de bases
(B) TYPE: nucléotide
(C) NOMBRE DE BRINS: simple
(D) CONFIGURATION: linéaire

(iv) ANTI-SENS: OUI

(xi) DESCRIPTION DE LA SEQUENCE: SEQ ID NO: 14:

TTGTAAGAAC TG 12

(2) INFORMATIONS POUR LA SEQ ID NO: 15:

(i) CARACTERISTIQUES DE LA SEQUENCE:
(A) LONGUEUR: 12 paires de bases
(B) TYPE: nucléotide
(C) NOMBRE DE BRINS: simple
(D) CONFIGURATION: linéaire

(iv) ANTI-SENS: OUI

(xi) DESCRIPTION DE LA SEQUENCE: SEQ ID NO: 15:

GAAGTGCTTC TC 12

(2) INFORMATIONS POUR LA SEQ ID NO: 16:

(i) CARACTERISTIQUES DE LA SEQUENCE:
(A) LONGUEUR: 12 paires de bases
(B) TYPE: nucléotide
(C) NOMBRE DE BRINS: simple
(D) CONFIGURATION: linéaire

(iv) ANTI-SENS: OUI

(xi) DESCRIPTION DE LA SEQUENCE: SEQ ID NO: 16:

GCCTCTTTTC AG 12

(2) INFORMATIONS POUR LA SEQ ID NO: 17:

(i) CARACTERISTIQUES DE LA SEQUENCE:
(A) LONGUEUR: 12 paires de bases
(B) TYPE: nucléotide
(C) NOMBRE DE BRINS: simple
(D) CONFIGURATION: linéaire

(iv) ANTI-SENS: OUI

(xi) DESCRIPTION DE LA SEQUENCE: SEQ ID NO: 17:

GGCATCTGTC AG 12

(2) INFORMATIONS POUR LA SEQ ID NO: 18:

(i) CARACTERISTIQUES DE LA SEQUENCE:
(A) LONGUEUR: 12 paires de bases
(B) TYPE: nucléotide
(C) NOMBRE DE BRINS: simple
(D) CONFIGURATION: linéaire

(iv) ANTI-SENS: OUI

(xi) DESCRIPTION DE LA SEQUENCE: SEQ ID NO: 18:

CGGCTGGCAT CC 12

(2) INFORMATIONS POUR LA SEQ ID NO: 19:

(i) CARACTERISTIQUES DE LA SEQUENCE:
(A) LONGUEUR: 12 paires de bases
(B) TYPE: nucléotide
(C) NOMBRE DE BRINS: simple

(D) CONFIGURATION: linéaire

(iv) ANTI-SENS: OUI

(xi) DESCRIPTION DE LA SEQUENCE: SEQ ID NO: 19:

GAACTGACAC AC 12

(2) INFORMATIONS POUR LA SEQ ID NO: 20:

(i) CARACTERISTIQUES DE LA SEQUENCE:
(A) LONGUEUR: 12 paires de bases
(B) TYPE: nucléotide
(C) NOMBRE DE BRINS: simple
(D) CONFIGURATION: linéaire

(iv) ANTI-SENS: OUI

(xi) DESCRIPTION DE LA SEQUENCE: SEQ ID NO: 20:

ACGATCACAT CA 12

(2) INFORMATIONS POUR LA SEQ ID NO: 21:

(i) CARACTERISTIQUES DE LA SEQUENCE:
(A) LONGUEUR: 12 paires de bases
(B) TYPE: nucléotide
(C) NOMBRE DE BRINS: simple
(D) CONFIGURATION: linéaire

(iv) ANTI-SENS: OUI

(xi) DESCRIPTION DE LA SEQUENCE: SEQ ID NO: 21:

ATCAACATGC AG 12

(2) INFORMATIONS POUR LA SEQ ID NO: 22:

(i) CARACTERISTIQUES DE LA SEQUENCE:
(A) LONGUEUR: 12 paires de bases
(B) TYPE: nucléotide
(C) NOMBRE DE BRINS: simple
(D) CONFIGURATION: linéaire

(iv) ANTI-SENS: OUI

(xi) DESCRIPTION DE LA SEQUENCE: SEQ ID NO: 22:

CTGCATATCG AC 12

(2) INFORMATIONS POUR LA SEQ ID NO: 23:

(i) CARACTERISTIQUES DE LA SEQUENCE:
(A) LONGUEUR: 12 paires de bases
(B) TYPE: nucléotide
(C) NOMBRE DE BRINS: simple
(D) CONFIGURATION: linéaire

(iv) ANTI-SENS: OUI

(xi) DESCRIPTION DE LA SEQUENCE: SEQ ID NO: 23:

[?]GTGCACCA GC 12

(2) INFORMATIONS POUR LA SEQ ID NO: 24:

(i) CARACTERISTIQUES DE LA SEQUENCE:
(A) LONGUEUR: 12 paires de bases
(B) TYPE: nucléotide
(C) NOMBRE DE BRINS: simple
(D) CONFIGURATION: linéaire

(iv) ANTI-SENS: OUI

(xi) DESCRIPTION DE LA SEQUENCE: SEQ ID NO: 24:

CTGCAGCCTC GG 12

(2) INFORMATIONS POUR LA SEQ ID NO: 25:

(i) CARACTERISTIQUES DE LA SEQUENCE:
(A) LONGUEUR: 12 paires de bases
(B) TYPE: nucléotide
(C) NOMBRE DE BRINS: simple
(D) CONFIGURATION: linéaire

(iv) ANTI-SENS: OUI

(xi) DESCRIPTION DE LA SEQUENCE: SEQ ID NO: 25:

ACTCTCTGGA CC 12

(2) INFORMATIONS POUR LA SEQ ID NO: 26:

(i) CARACTERISTIQUES DE LA SEQUENCE:
(A) LONGUEUR: 12 paires de bases
(B) TYPE: nucléotide
(C) NOMBRE DE BRINS: simple
(D) CONFIGURATION: linéaire

(iv) ANTI-SENS: OUI

(xi) DESCRIPTION DE LA SEQUENCE: SEQ ID NO: 26:

CAAGAATCCT CG 12

(2) INFORMATIONS POUR LA SEQ ID NO: 27:

(i) CARACTERISTIQUES DE LA SEQUENCE:
(A) LONGUEUR: 12 paires de bases
(B) TYPE: nucléotide
(C) NOMBRE DE BRINS: simple
(D) CONFIGURATION: linéaire

(iv) ANTI-SENS: OUI

(xi) DESCRIPTION DE LA SEQUENCE: SEQ ID NO: 27:

ACATCTGCTC TG 12

(2) INFORMATIONS POUR LA SEQ ID NO: 28:

(i) CARACTERISTIQUES DE LA SEQUENCE:
(A) LONGUEUR: 12 paires de bases
(B) TYPE: nucléotide
(C) NOMBRE DE BRINS: simple
(D) CONFIGURATION: linéaire

(iv) ANTI-SENS: OUI

(xi) DESCRIPTION DE LA SEQUENCE: SEQ ID NO: 28:

TCTGTACTCG TC 12

(2) INFORMATIONS POUR LA SEQ ID NO: 29:

(i) CARACTERISTIQUES DE LA SEQUENCE:
(A) LONGUEUR: 12 paires de bases
(B) TYPE: nucléotide
(C) NOMBRE DE BRINS: simple

(D) CONFIGURATION: linéaire

(iv) ANTI-SENS: OUI

(xi) DESCRIPTION DE LA SEQUENCE: SEQ ID NO: 29:

CTACAGTACA GC 12

(2) INFORMATIONS POUR LA SEQ ID NO: 30:

(i) CARACTERISTIQUES DE LA SEQUENCE:
(A) LONGUEUR: 15 paires de bases
(B) TYPE: nucléotide
(C) NOMBRE DE BRINS: simple
(D) CONFIGURATION: linéaire

(iv) ANTI-SENS: OUI

(xi) DESCRIPTION DE LA SEQUENCE: SEQ ID NO: 30:

CCCGTCGTCC ATAGG 15

(2) INFORMATIONS POUR LA SEQ ID NO: 31:

(i) CARACTERISTIQUES DE LA SEQUENCE:
(A) LONGUEUR: 18 paires de bases
(B) TYPE: nucléotide
(C) NOMBRE DE BRINS: simple
(D) CONFIGURATION: linéaire

(iv) ANTI-SENS: OUI

(xi) DESCRIPTION DE LA SEQUENCE: SEQ ID NO: 31:

CCCGTCGTCC ATAGGGGA 18

(2) INFORMATIONS POUR LA SEQ ID NO: 32:

(i) CARACTERISTIQUES DE LA SEQUENCE:
(A) LONGUEUR: 21 paires de bases
(B) TYPE: nucléotide
(C) NOMBRE DE BRINS: simple
(D) CONFIGURATION: linéaire

(iv) ANTI-SENS: OUI

(xi) DESCRIPTION DE LA SEQUENCE: SEQ ID NO: 32:

CCCGTCGTCC ATAGGGGACG C 21

(2) INFORMATIONS POUR LA SEQ ID NO: 33:

(i) CARACTERISTIQUES DE LA SEQUENCE:
(A) LONGUEUR: 15 paires de bases
(B) TYPE: nucléotide
(C) NOMBRE DE BRINS: simple
(D) CONFIGURATION: linéaire

(iv) ANTI-SENS: OUI

(xi) DESCRIPTION DE LA SEQUENCE: SEQ ID NO: 33:

[?]ACGTGACT GTTTC 15

(2) INFORMATIONS POUR LA SEQ ID NO: 34:

(i) CARACTERISTIQUES DE LA SEQUENCE:
(A) LONGUEUR: 18 paires de bases
(B) TYPE: nucléotide
(C) NOMBRE DE BRINS: simple
(D) CONFIGURATION: linéaire

(iv) ANTI-SENS: OUI

(xi) DESCRIPTION DE LA SEQUENCE: SEQ ID NO: 34:

GGACGTGACT GTTTCCAC 18

(2) INFORMATIONS POUR LA SEQ ID NO: 35:

(i) CARACTERISTIQUES DE LA SEQUENCE:
(A) LONGUEUR: 21 paires de bases
(B) TYPE: nucléotide
(C) NOMBRE DE BRINS: simple
(D) CONFIGURATION: linéaire

(iv) ANTI-SENS: OUI

(xi) DESCRIPTION DE LA SEQUENCE: SEQ ID NO: 35:

GGACGTGACT GTTTCCACTG A 21

(2) INFORMATIONS POUR LA SEQ ID NO: 36:

(i) CARACTERISTIQUES DE LA SEQUENCE:
(A) LONGUEUR: 24 paires de bases
(B) TYPE: nucléotide
(C) NOMBRE DE BRINS: simple
(D) CONFIGURATION: linéaire

(iv) ANTI-SENS: OUI

(xi) DESCRIPTION DE LA SEQUENCE: SEQ ID NO: 36:

GGACGTGACT GTTTCCACTG AGTC 24

## Revendications

1. Un oligonucléotide hybridable sélectivement avec un gène ou les produits de transcription pour les sous-unités des isoprényl protéine transférases.

2. Un oligonucléotide comprenant au moins un fragment de l'une des séquences sélectionnées parmi les séquences SEQ ID No. 1 à SEQ ID No. 36.

3. Un oligonucléotide selon la revendication 1 ou 2, hybridable avec un gène ou les produits de transcription des sous-unités α et / ou β des isoprényl protéine transférases.

4. Un oligonucléotide selon la revendication 1 ou 3, dans lequel l'isoprényl protéine transférase est choisi parmi la farnésyl-protéine transférase, la géranyl-géranyl-protéine transférase de type 1 et la géranyl-géranyl-protéine transférase de type 2.

5. Un oligonucléotide selon l'une quelconque des revendications précédentes, qui comprend de 2 à 50 unités.

6. Un oligonucléotide selon la revendication 5, qui comprend de 8 à 35 unités.

7. Un oligonucléotide selon la revendication 6, qui comprend de 10 à 25 unités.

8. Un oligonucléotide selon l'une quelconque des revendications précédentes, dont le squelette est modifié.

9. Un oligonucléotide selon la revendication 8, dans lequel la base nucléotidique est modifiée.

10. Un oligonucléotide selon la revendication 9, dans lequel la base nucléotidique modifiée présente la conformation α-anomer.

11. Un oligonucléotide selon la revendication 8, dans lequel au moins un des groupes de liaison est modifié.

12. Un oligonucléotide selon la revendication 8, dans lequel soit la position 5' ou la position 3', soit les positions 5' et 3' est modifiée.

13. Un oligonucléotide selon la revendication 12, dans lequel soit la position 5' ou la position 3', soit les positions 5' et 3' est modifiée par la substitution d'un groupe hydrophobe ou protecteur.

14. Compositions thérapeutiques contenant comme principe actif au moins un oligonucléotide selon l'une quelconque des revendications précédentes, mélangé avec un excipient et / ou support pharmaceutiquement acceptable, selon le mode d'administration choisi.

15. Utilisation d'une composition selon la revendication 14, pour une méthode de traitement ou thérapie des maladies du corps humain ou animal dans lesquelles la prolifération cellulaire est anormale et / ou incontrôlée.
